(19) Europäisches Patentamt
European Patent Office
Office européen des brevets

(11) **EP 4 386 389 B1**

(12) **EUROPEAN PATENT SPECIFICATION**

(45) Date of publication and mention
of the grant of the patent:
**01.04.2026 Bulletin 2026/14**

(21) Application number: **22213596.4**

(22) Date of filing: **14.12.2022**

(51) International Patent Classification (IPC):
**G01N 35/10** (2006.01)   **G01N 13/02** (2006.01)
**G01N 11/08** (2006.01)

(52) Cooperative Patent Classification (CPC):
**G01N 35/1016; G01N 11/08; G01N 13/02;**
G01N 2013/0208; G01N 2013/0241;
G01N 2013/0266; G01N 2035/103;
G01N 2035/1062

(54) **DETERMINING PHYSICAL PARAMETERS OF A LIQUID**

BESTIMMUNG PHYSIKALISCHER PARAMETER EINER FLÜSSIGKEIT

DÉTERMINATION DE PARAMÈTRES PHYSIQUES D'UN LIQUIDE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**19.06.2024 Bulletin 2024/25**

(60) Divisional application:
**25223809.2 / 4 692 757**

(73) Proprietor: **Tecan Trading AG
8708 Männedorf (CH)**

(72) Inventors:
• **KELLER, Michael
8494 Bauma (CH)**
• **GEIGES, Thomas
8708 Männedorf (CH)**
• **VILAJ, Volfgang
8707 Uetikon am See (CH)**

(74) Representative: **Sykora & König Patentanwälte
PartG mbB
Maximilianstraße 2
80539 München (DE)**

(56) References cited:
**US-A1- 2013 045 498     US-A1- 2022 026 455
US-A1- 2022 196 696**

**Description**

FIELD OF THE INVENTION

The invention relates to a method, a computer program, a computer-readable medium and a control device for determining physical parameters of a liquid. Furthermore, the method relates to a laboratory automation device.

BACKGROUND OF THE INVENTION

Laboratory automation devices are used for automating tasks of a laboratory assistant, which, for example, tests a patient for specific diseases. Usually, a sample of the patient's blood, urine, stool, etc. is taken and analysed by means of a biochemical procedure. Such a procedure consists in various operations like adding substances, incubating, separating, etc. and a measurement process which quantitatively or qualitatively measures the amount or presence of a substance indicating the specific disease.

Sometimes laboratory automation devices need to deal with unknown liquids or with liquids such as blood, which have varying physical properties such as density and viscosity. In these cases, it may be that the user of the laboratory automation device wants to know the properties of the liquid. It also may be that the physical properties have to be determined to calculate and set the liquid class parameters such as accuracy correction, asp/dsp speeds, etc. A liquid class for a specific liquid is used for controlling the laboratory automation device, when the laboratory automation device processes this liquid.

US 2022/026455 A1 describes a method for classifying liquid handling procedures with a neuronal network. From a measured pressure curve physical parameters, such as density, viscosity, surface tension and wettability of the pipette tip, are determined.

DESCRIPTION OF THE INVENTION

It is an objective of the invention to automate and simplify the determination of physical parameters of liquids used in the context of laboratory automation.

This objective is achieved by the subject-matter of the independent claims. Further exemplary embodiments are evident from the dependent claims and the following description.

A first aspect of the invention relates to a method for determining physical parameters of a liquid to be aspirated and/or dispensed by a laboratory automation device. The method may be performed automatically by the laboratory automation device and in particular a control device of the laboratory automation device. A control device of a laboratory automation device, which control device is adapted for performing the method, also is a further aspect of the invention.

According to an embodiment of the invention, the method comprises: pickup of a pipette with the laboratory automation device: A pipetting arm may take the pipette. The pipette may be disposable. A disposable pipette may be called "pipette tip", however, in the following, the end of the pipette with the orifices will be called pipette tip.

The method further comprises: lowering the pipette into a sample container with the laboratory automation device, the sample container containing the liquid. The liquid may be an unknown liquid, for which the physical parameters are to be determined.

The method further comprises: aspirating and dispensing air and liquid with the laboratory automation device in such a way, that a liquid level in the pipette solely rises in a first step and solely lowers in a second step, such that an interior surface of the pipette is wetted with liquid solely one time. For determining the physical properties with high accuracy, they are determined in one pass, where the liquid level in the pipette solely rises once and lowers once. This may mean that in the first step, when the pipette tip is within the liquid, solely an underpressure is generated in the pipette. In the second step, then solely an overpressure is generated.

The method further comprises: during aspirating and dispensing air and liquid, measuring a pressure curve in the pipette and determining the physical parameters from the pressure curve, the physical parameters comprising at least one of a wetting angle and a viscosity. The pressure curve may be measured with a pressure sensor of the laboratory automation device, which pressure sensor may be connected to the interior of the pipette. For example, the pressure sensor may be a part of the pipetting arm or the pump generating the pressure in the pipette.

The physical parameters may be determined from a set of equations, which models the liquid and the pipette. Specific pressure values at different times of the pressure curve, such as specific peaks, may be input into the equations to calculate the physical parameters. The equations furthermore may comprise parameters, which model the pipette, such as a geometric shape of the pipette, a level at which a cylindrical part of the pipette starts, an orifice radius, an opening angle of a cone providing the orifice, etc. These pipette parameters may be fixed and/or stored in the control device, when a normed pipette tip is used.

It has to be noted that the physical parameters already may be determined during the measurement of the pressure curve. However, also the pressure curve first may be recorded completely and, for example, stored in the control device. After that, the pressure curve may be evaluated and the physical parameters may be determined.

According to an embodiment of the invention, the pipette is unused and has not been wetted with a liquid before. A wetting of the interior surface of the pipette before this method step may influence the determination of physical parameters, such as the surface tension, volatility, wetting angle and viscosity. Starting with an unused pipette will increase the accuracy of these parameters.

According to an embodiment of the invention, the method further comprises: before lowering the pipette into the sample container for a first time: aspirating and/or dispensing air into the pipette; and determining a filter resistance of a filter inside the pipette from the pressure curve during aspirating and/or dispensing air. The filter resistance of a possible filter in the pipette is determined in the beginning of the method without immersing the pipette tip into the liquid. The filter resistance can be determined from a flowrate, which may be provided by the pump, and a pressure difference at the beginning and end of the aspiration and of the dispensing.

According to an embodiment of the invention, the method further comprises: determining an aspirating-dispensing asymmetry from the measured pressure values during aspirating and dispensing air. An aspiration filter resistance during aspiration and a dispensing filter resistance during dispensing can be determined and compared with each other. A dispensing asymmetry higher than a threshold may indicate an error or a problem with the pipette.

According to an embodiment of the invention, the method further comprises: after lowering the pipette into the sample container for the first time: dispensing air into the liquid and generating at least one bubble in the liquid; determining a surface tension of the liquid from the measured pressure curve during generating the bubble. Possibly after the filter resistance determination and/or before aspirating and dispensing air and liquid in such a way, that a liquid level in the pipette solely rises in a first step and solely lowers in a second step, one or more bubbles may be generated in the liquid. When the bubbles leave the pipette tip, a maximal pressure is related to the surface tension of the liquid and the surface tension can be determined from the generated pressure curve.

The test, whether the pipette tip has been lowered into the liquid, i.e. that the orifice is below the liquid level, may be done by measuring an electric capacitance between pipette tip and liquid. Capacitance liquid level detection may allow to accurately control the submerge of the pipette tip.According to an embodiment of the invention, the method further comprises: after generating the one or more bubbles, withdrawing the pipette from liquid and dispensing air to remove liquid from the pipette tip. To enhance the accuracy of the following measurements, the pipette tip and/or the orifice in the pipette may be freed from liquid by dispensing air.

According to an embodiment of the invention, the method further comprises: waiting without aspirating and dispensing for a waiting time period, while liquid is in the pipette; and determining a volatility of the liquid from the measured pressure curve during the waiting time period. This may be done after filter resistance determination and/or surface tension determination and/or at the beginning of aspirating and dispensing air and liquid in such a way, that a liquid level in the pipette solely rises in a first step and solely lowers in a second step. Before waiting for the time period, some liquid has to be aspirated and/or during waiting, no aspirating and dispensing should be performed. During the waiting, dissolved gas and/or vapor may leave the liquid inside the pipette causing a pressure change. From this pressure change and a known amount of liquid in the pipette, the volatility may be determined. The known amount of liquid in the pipette can be determined from the flowrate and time of aspiration and the pressure in the tip after aspiration before starting the waiting time period.

According to the invention, the method further comprises: determining the wetting angle of the liquid from the pressure curve during a time period with continuously raising and/or lowering of the liquid level in the pipette. This may be done during aspirating and dispensing air and liquid in such a way, that a liquid level in the pipette solely rises in a first step and solely lowers in a second step. The wetting angle can be determined from the pressure curve during a time with continuously raising or lowering the liquid level. The lowering and/or raising may be done with constant speed of the liquid level.

According to the invention, an advancing wetting angle of the liquid is determined for a raising liquid level and/or a receding wetting angle of the liquid is determined for a lowering liquid level. From these two wetting angles, a mean value may be determined. A hysteresis of the two wetting angles may be determined from the difference of the two wetting angles.

According to an embodiment of the invention, the method further comprises: determining a viscosity of the liquid from the pressure curve during a time period with continuously raising and/or lowering of the liquid in the pipette. This may be done during aspirating and dispensing air and liquid in such a way, that a liquid level in the pipette solely rises in a first step and solely lowers in a second step. The viscosity can be determined from the pressure curve during a time with continuously raising or lowering the liquid level. The lowering and/or raising may be done with constant speed of the liquid level. Dispensing of the liquid also may be done, such that the liquid is dispensed in air.

According to an embodiment of the invention, the method further comprises: determining a density of the liquid from the pressure curve from two pressures of the pressure curve at two times with two different known liquid levels. The density may be determined from the weight of the difference volume of the liquid between the two liquid levels. This weight depends on the pressure difference at these two liquid levels. The density may also be determined by analyzing the slope of the pressure curve during aspiration and/or dispensing, if other effects such as filter resistance, contact angle, etc., are subtracted.

According to an embodiment of the invention, the physical parameters are determined from a set of equations modelling the liquid and the pipette. The equations may be seen as a physical model of the relevant parts of the laboratory automation system. The set of equations comprises the physical parameters of the liquid and parameters of the pipette. Such pipette parameters may comprise a geometric shape of the pipette, the start of a cylindrical part of the pipette, the diameter of the cylindrical part, an orifice radius of the pipette tip, a tip opening angle of the pipette tip and/or its orifice, etc.

The pipette parameters may be fixed and/or stored in the control device. In this case, measured tips may be used to reduce the tolerances of physical parameter determination. However, it is also possible to determine the pipette parameters with the same or an analogous method, however with a known liquid, from which the physical parameters are known. The pipette parameters can be determined from the pressure curve with the help of the known liquid parameters of the known liquid, such as water.

According to an embodiment of the invention, the liquid (described above) is an unknown liquid, the pipette is a second pipette and the sample container is a second sample container.

All the parameters may be determined by performing the method twice, once with a known, reference liquid (such as water) and then redoing the method with the unknown liquid for which we are interested in the properties. This may be possible as pipettes from the same lot and in particular from the same cavity of the mold may have exactly the same geometry.

A first pressure curve for the known liquid and a second pressure curve for the unknown liquid may be determined and/or measured. All unknown parameters may be determined from both pressure curves. It has to be noted that also firstly, the second pressure curve with the unknown liquid and after, that the first pressure curve with the known liquid may be determined and/or measured.

According to an embodiment of the invention, the method further comprises: pickup of a first pipette with the laboratory automation device, the first pipette being of equal structural form as the second pipette; lowering the first pipette into a first sample container with the laboratory automation device, the first sample container containing a known liquid with known physical parameters; aspirating and dispensing air and the known liquid with the laboratory automation device in such a way, that a liquid level in the first pipette solely rises in a first step and solely lowers in a second step, such that an interior surface of the first pipette is wetted with the known liquid solely one time; during aspirating and dispensing air and the known liquid, measuring a pressure curve in the first pipette and determining parameters of the first and second pipette from the pressure curve.

The first pressure curve for the known liquid may be determined with the same aspiration and dispensing steps as the pressure curve for the unknown liquid.

The determined physical parameters of the liquid may be output to a user of the laboratory automation device, for example for planning an assay procedure with the liquid.

According to an embodiment of the invention, the method further comprises: storing the physical parameters in a liquid class for the liquid; and optionally performing an assay procedure with the liquid using the liquid class. A predefined liquid class may be selected and the physical parameters there may be adjusted to the determined values. Also a new liquid class may be defined with the determined values.

It also may be that a liquid class for the liquid is stored in the control device. A liquid class is a data structure used by the control device and/or the laboratory automation device for handling the liquid. For example, the liquid class sets, how fast the liquid is aspirated and dispensed during specific steps of an assay procedure. Another example for a parameter in the liquid class is an accuracy correction or the wait time after aspiration. An assay procedure may be a predefined set of operations that are automatically performed by the laboratory automation device for processing the liquid.

A further aspect of the invention relates to a computer program for determining physical parameters of a liquid, which computer program, when being executed by a processor, is adapted to carry out the steps of the method as described above and below. The computer program may be executed in a computing device, such as a control device of the laboratory automation device and/or a PC, which may be communicatively interconnected with the laboratory automation device. It also is possible that the method is performed by an embedded microcontroller of the laboratory automation device.

A further aspect of the invention relates to a computer-readable medium, in which such a computer program is stored. A computer-readable medium may be a floppy disk, a hard disk, an USB (Universal Serial Bus) storage device, a RAM (Random Access Memory), a ROM (Read Only Memory), an EPROM (Erasable Programmable Read Only Memory) or a FLASH memory. A computer-readable medium may also be a data communication network, e.g. the Internet and/or a cloud storage, which allows downloading a program code. In general, the computer-readable medium may be a non-transitory or transitory medium.

A further aspect of the invention relates to a laboratory automation device.

According to an embodiment of the invention, the laboratory automation device comprises a pipetting arm for carrying a pipette; a pressure device for changing a pressure in a volume connected to the pipette for aspirating and dispensing a liquid medium in the pipette; a pressure sensor for pressure measurements in the volume connected to the pipette and a control device for controlling the pump and the pipetting arm and for receiving a pressure signal from the pressure sensor, wherein the control device is adapted for performing the method as described above and below.

It has to be understood that features of the method as described in the above and in the following may be features of the control device, the computer program and the computer-readable medium as described in the above and in the following and vice versa.

These and other aspects of the invention will be apparent from and elucidated with reference to the embodiments described hereinafter.

BRIEF DESCRIPTION OF THE DRAWINGS

Below, embodiments of the present invention are described in more detail with reference to the attached drawings.

Fig. 1 schematically shows a laboratory automation device according to an embodiment of the invention.

Fig. 2 shows a diagram with pressure over time produced during the execution of the method described herein.

The reference symbols used in the drawings, and their meanings, are listed in summary form in the list of reference symbols. In principle, identical parts are provided with the same reference symbols in the figures.

DETAILED DESCRIPTION OF EXEMPLARY EMBODIMENTS

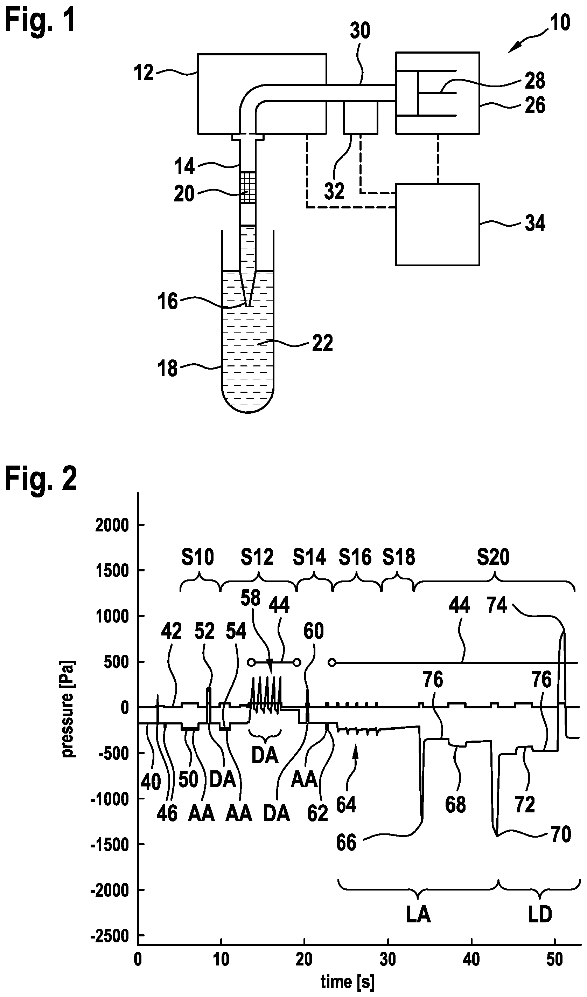

Fig. 1 schematically shows a laboratory automation device 10, which comprises an automatically movable pipetting arm 12 to which a pipette 14 is attached. As shown in Fig. 1, the pipette tip 16 of the pipette 14 may be lowered into a sample container 18 via the movable pipetting arm 12.

The pipetting arm 12 may move the pipette 14 and the pipette tip 16 in three dimensions, may lower the pipette tip 16 into the sample container 18 and may retract the pipette tip 16 therefrom.

The pipette 14 may contain a filter 20, which prevents entering substances from the pipette 14 into the remaining parts of the laboratory automation device 10.

In the sample container 18, a liquid 22 is contained. When the pipette tip 16 is immersed in the liquid in the sample container 18, the liquid can be aspirated into the pipette 14.

The laboratory automation device 10 comprises a pump 26, which is connected via a hose 30 with the pipette 14. With the pump 26, a pressure may be applied to the hose 30 and to the pipette 14, or more general to a volume 30 connected with the pipette 14, which causes the pipette 14 to aspirate or dispense the liquid 22 or air. For example, the pump 26 comprises a plunger 28, which is moved for generating underpressure and overpressure in the hose 30 and the pipette 14. Any other kind of pumps, e.g. peristaltic pumps, or in general any source of vacuum and pressure may be used. The source of vacuum and pressure may be controlled via valves.

A pressure sensor 32, which may be attached to the hose 30 and/or the pipette 14, is adapted for measuring a pressure in the hose 30 and/or the pipette 14.

A control device 34 of the laboratory automation device 10, which may be a part of the laboratory automation device 10 or connected thereto, may control the pipetting arm 12, the pump 26 and may receive a pressure signal from the pressure sensor 32.

Fig. 2 shows a diagram with the pressure 40 measured by the sensor 32 over time. During aspirating and dispensing air and liquid 22 performed by the laboratory automation device 10 in the following, a pressure curve 40 is measured in the pipette 14 with the sensor 32.

From the pressure curve 40, physical parameters of the liquid 22, such as a surface tension, a wetting angle, a viscosity, etc., can be determined. This may be done by the control device 34 during the steps described in the following or after the complete pressure curve 40 has been measured.

A further graph shows the activity 42 of the pump 26. The activity 42 indicates, whether the pump 26 is operating (upper level of graph) or not (lower level of graph).

The two lines 44 show, when the pipette tip 16 is immersed in the liquid 22. Otherwise, the pipette tip 16 is surrounded by air above the liquid 22.

In region "AA", air is aspirated with the pipette 14 through the pipette tip 16. In "AD", air is dispensed. In "LA", liquid 22 is aspirated. In "LD", liquid 22 is dispensed. In general, aspiration of air and/or liquid is caused by lowering the pressure 40 in the pipette 14 and dispensing is caused by raising the pressure 40.

During the following test sequence, it is important that the interior surface of the pipette 14 is wetted solely one time. The test sequence is chosen, such that firstly the liquid level inside the pipette is only raising ("LA") and after that, the liquid level is solely falling ("LD"). Air and liquid 22 is aspirated and dispensed with the laboratory automation device 10 in such a way, that a liquid level in the pipette 14 solely rises in a first step ("LA") and solely lowers in a second step ("LD"), such that an interior surface of the pipette 14 is wetted with liquid solely one time.

Furthermore, at the beginning of the following test sequence it is important that an unused pipette 14 is used, i.e. that the pipette 14 is empty and that no liquid 22 is in the pipette 14 and in particular was not in the pipette 14, i.e. was not wetted with a liquid 22 before. In such a way it is assured that no liquid 22 is still wetting the interior surface of the pipette 14 and that there is solely pure air inside without any remaining such as water vapor or other gases.

In the beginning, the pipette tip 16 is outside the liquid 22. The sensor 32 needs not be calibrated to 0 and therefore, at the beginning, the pressure 40 is not shown at zero level in Fig. 2. The pressure, when the pipette tip is empty and contains no liquid, as well as no aspiration or dispensing is done and the pipette tip is in air will be called neutral pressure level $p_0$.

The first peaks 46 in the pressure curve 40 are caused by picking up the pipette 16 with the arm 12.

In step S10, the filter 20 is tested with air. Air is aspirated into the pipette 14 (peak 50), dispensed (peak 52). From the pressure difference, the filter resistance can be calculated by dividing the flowrate through the pressure difference. The flowrate may be determined from a movement difference of the plunger 28, or more general from the speed of the pump 26. In particular, a first filter resistance $r_1$ is calculated from the flowrate $q_1$ and pressure difference $\Delta p_1$ between 50 and the neutral pressure level.

$$r_1 = \frac{\Delta p_1}{q_1}$$

Additionally, a second filter resistance $r_2$ is calculated from the flowrate $q_2$ and pressure difference $\Delta p_2$ between 52 and the neutral pressure level.

$$r_2 = \frac{\Delta p_2}{q_2}$$

Before lowering the pipette 14 into the sample container 18 for a first time, air is aspirated and dispensed into the pipette 14. A filter resistance $r_1$, $r_2$ of the filter 20 inside the pipette 14 is determined from the pressure curve 40 during this aspiration and/or dispensing procedure.

An aspirating-dispensing asymmetry may be determined from the measured pressure values during aspirating and dispensing air in this step, in particular from the first filter resistance $r_1$ and the second filter resistance $r_2$. For example, when their difference is greater than a threshold, an asymmetry is detected and a problem with the filter 22 and/or the procedure may be identified. An average filter resistance $r$ may be determined as average of the first and second filter resistance $r_1$, $r_2$.

In step S12, firstly air is aspirated again into the pipette (peak 54). This is in preparation for the following, as air needs to be aspirated into a plunger based device 10 to be able to dispense air. However, also other types of pumps may be used. the pipette tip 16 is lowered into the liquid 22. After that, the pressure 40 is raised in the pipette 14 and bubbles are generated at the pipette tip 16. When they grow beyond the radius of the pipette orifice, the pressure drops, resulting in the peaks 58 and these bubbles detach from the pipette tip 16. From the bubble pressure $p_b$ at each peak 58, the surface tension $\sigma$ of the liquid 22 can be calculated.

$$\sigma = \frac{r_0}{2}(p_b - g * s * \rho)$$

Where $r_0$ is the orifice radius of the pipette tip 16, $g$ is the gravity acceleration, $s$ is the submerge of the tip orifices (usually 1-2 mm) and $\rho$ is the density of the liquid 22. Here, it is firstly assumed that $\rho$ is the density of water. If the later determined density of the liquid 22 deviates for example less than 20% of that from water, this has enough accuracy. Otherwise, the surface tension $\sigma$ and the following values may have to recalculated with the later determined density.

The bubble pressure

$$p_b = p_{meas} - p_0 - p_{Filter}$$

is the maximum of the pressure $p_{meas}$ at the respective peak 58, when the bubble leaves the tip 16, from which the neutral pressure level $p_0$ and the filter pressure $p_{Filter}$ are subtracted. The filter pressure $p_{Filter} = l_v * r$ is the product of flowrate $l_v$ and filter resistance $r$. The flowrate $l$ may be determined from the volume change caused by the plunger 28 or more general from the activity of the pump 26.

After lowering the pipette 14 into the sample container 18 for the first time, air is dispensed into the liquid 22 and at least one bubble is generated in the liquid 22. A surface tension $\sigma$ of the liquid 22 is then determined from the measured pressure curve 40 during generating the one or more bubbles.

From the values of the surface tension $\sigma$ for each bubble and/or peak 58, also a mean of the surface tension can be calculated. A difference of the surface tension per bubble with the mean surface tension of more than a threshold may indicate a problem with the procedure.

In step S14, the pipette tip 16 is retreated from the liquid 22. Remaining liquid 22 and in particular a potential drop below the tip is removed from the tip by blowing air from the tip (peak 60). After generating the one or more bubbles, the pipette 14 is withdrawn from the liquid 22 and air is dispensed to remove remaining liquid 22 from the pipette tip 16.

Air is then aspirated again (peak 62), to fill up the system behind the pipette 14 with air.

In step S16, the pipette tip 16 is lowered into the liquid 22 again and with several small aspirations (peaks 64) it is tested, when liquid 22 enters the pipette 14. This gives a first estimate of the wetting angle $\alpha_{ws}$ (in theory the static wetting angle), to decide if the wetting angle is below 90 degree or above and if above a first estimate. If the pressure rises right after the pipette tip 16 has touched the liquid surface, this means that liquid 22 went into the pipette tip 16 and that the wetting angle is below 90°. If no liquid 22 directly went into the pipette tip 16, the pressure required for liquid 22 to come into the pipette tip 22 gives a first estimate of the wetting angle. In the present example, at the first aspiration in step S16 the pressure just drops, which means no liquid 22 is in the pipette tip 22. After the second aspiration is step S16 the pressure rises again after the drop which means that liquid 22 came in. The wetting angle $\alpha_w$ can be determined via

$$\alpha_w = \mathrm{acos}\left(p_t * \frac{r_0}{2\sigma}\right) - \alpha_t$$

where $p_t$ is the measured pressure with hydrostatic pressure subtracted after the last step of S16, $r_0$ is the orifice radius, $\sigma$ is the surface tension, $\alpha_t$ is the tip angle, i.e. the opening angle of the pipette tip 16 at the orifice. This formula also may be used for the wetting angle determination with respects to peaks 68 and 72 below.

In step S18, the raise in pressure caused by gassing out of the liquid 22 is determined, i.e. its volatility $v$.

The formula for the ideal gas law p*V=n*R*T (also called general gas equation) can be used to relate the amount of substance (gas) in mol to the pressure in Pascal. Similarly, the change rate of amount of substance or volatility $v$ in mol/s can be related to the change rate of pressure in Pascal per second.

It is waited without aspirating and dispensing for a specific waiting time period without active pressure change. A volatility $v$ of the liquid 22 is determined from the measured pressure curve 40 during the time period.

In step S20, first an amount of liquid 22 is aspirated into the pipette 14 (peak 66), until the liquid level reaches the section of the pipette 16, which is close to a cylindrical shape. The position of the liquid level may be determined from the flowrate of the pump 26 and a shape model of the pipette 14 and/or the aspirated volume, which may be determined from the movement of the plunger 28. A small, deviation comes from the volatility v of the liquid 22, which may be corrected, when the volatility has been determined (see below).

Then, liquid 22 is aspirated causing peak 68, from which the advancing wetting angle of the liquid $\alpha_{wa}$ can be calculated (see formula above).

After that, the liquid 22 is aspirated with a higher speed and/or higher flowrate causing peak 70. From this, a first viscosity $\mu_1$ of the liquid 22 can be calculated. At this time, the liquid level 22 has reached a second defined value.

For determining the viscosity, the Hagen-Poisseuille formula may be used, which has to be integrated along the shape of the pipette 14, in particular its conical and cylindrical sections. For a cylindrical section, the result is

$$p_{HP} = \frac{8 * \mu_1 * L * I_v}{\pi * r_0{}^4}$$

where $p_{HP}$ is the pressure during a constant flowrate, L is the length of the cylindrical section, $r_0$ is the radius of the cylindrical section, and $I_v$ is the flowrate.

Then, liquid 22 is dispensed causing peak 72, from which the receding wetting angle of the liquid $\alpha_{wr}$ can be calculated (see formula above).

After that, the liquid 22 is dispensed with a higher speed and/or flowrate causing peak 74. From this, a second viscosity $\mu_2$ of the liquid 22 can be calculated. From the first viscosity $\mu_1$ and the second viscosity $\mu_2$, a mean viscosity $\mu$ of the liquid 22 can be calculated.

In general, the wetting angle $\alpha_{wa}$, $\alpha_{wr}$ of the liquid 22 is determined from the pressure curve 40 during a time period with continuously raising and/or lowering of the liquid level in the pipette 14. In particular, the advancing wetting angle $\alpha_{wa}$ is determined for a raising liquid level and/or the receding wetting angle $\alpha_{wr}$ is determined for a lowering liquid level.

Furthermore, the viscosity $\mu_1$, $\mu_2$ of the liquid 22 is determined from the pressure curve 40 during a time period with continuously raising and/or lowering of the liquid 22 in the pipette 14.

From the advancing wetting angle $\alpha_{wa}$ and the receding wetting angle $\alpha_{wr}$, the wetting angle hysteresis can be calculated as the difference of these two angles.

From the pressure difference $\Delta p$ between the points 76, where the first liquid level and the second liquid level have been reached, the density $\rho$ of the liquid 22 can be calculated by

$$\Delta p = \rho g h$$

where $g$ is the gravity acceleration and h is the difference between the first and second liquid level.

The density $\rho$ of the liquid 22 is determined from the pressure curve 40 from two pressure values 76 of the pressure curve 40 at two times with two different known liquid levels.

As described above, the physical parameters, such as surface tension $\sigma$, volatility v, wetting angle $\alpha_{wa}$, $\alpha_{wr}$, viscosity $\mu_1$, $\mu_2$ and density $\rho$, are determined from a set of equations modelling the liquid 22 and the pipette 14. Examples of such equations are provided above. It can be seen that the set of equations comprises the physical parameters of the liquid 22 and additionally parameters of the pipette 14, such as the orifice radius $r_0$.

The pipette parameters may be stored in the control device 34 for specific types of pipettes and/or may be measured manually before the physical parameters are determined.

It is also possible that the pipette parameters are determined in first pass of performing the method, however, with a liquid, for which the physical parameters are known. This may be done with a pipette of the same type as the pipette, which is used for determining the physical parameters. With the aid of the same set of equations, the pipette parameters then can be determined. The first pass with the known liquid may contain the same aspiration and dispensing steps as the second pass with the unknown liquid, in which the physical parameters of the unknown liquid are determined. The first pass may be performed before or after the second pass.

LIST OF REFERENCE SYMBOLS AND NOMENCLATURE

| 10 | laboratory automation device |
| 12 | pipetting arm |
| 14 | pipette |
| 16 | pipette tip |
| 18 | sample container |
| 20 | filter |
| 22 | liquid |
| 26 | pump |
| 28 | plunger |
| 30 | hose / volume |
| 32 | pressure sensor |
| 34 | control device |
| 40 | pressure curve / pressure signal |
| 42 | pump activity |
| 44 | tip immersion |
| AA | air aspiration |
| AD | air dispense |
| LA | liquid aspiration |
| LD | liquid dispense |
| 46 | picking up pipette |
| 50 | air aspiration |
| 52 | air dispense |
| 54 | air aspiration |
| 58 | bubble generation |
| 60 | liquid remove |
| 62 | air refill |
| 64 | small liquid aspirations |
| 66 | fill up to cylindrical section |
| 68 | slow aspiration for advancing wetting angle |
| 70 | fast aspiration for viscosity |
| 72 | slow dispense for receding wetting angle |
| 74 | fast dispense for viscosity |
| 76 | First liquid level and the second liquid level for determination of density |

**Claims**

1. A method for determining physical parameters of a liquid (22) to be aspirated and/or dispensed by a laboratory automation device (10), the method comprising:

   pickup of a pipette (14) with the laboratory automation device (10);
   lowering the pipette (14) into a sample container (18) with the laboratory automation device (10), the sample container (18) containing the liquid (22);
   aspirating and dispensing air and liquid (22) with the laboratory automation device (10) in such a way, that a liquid level in the pipette (14) solely rises in a first step and solely lowers in a second step, such that an interior surface of the pipette (14) is wetted with liquid solely one time;
   during aspirating and dispensing air and liquid (22), measuring a pressure curve (40) in the pipette (14) and determining the physical parameters from the pressure curve (40), the physical parameters comprising a wetting angle of the liquid (22) and optionally a surface tension and/or optionally a viscosity;
   wherein the wetting angle of the liquid (22) is determined from the pressure curve (40) during a time period with continuously raising and/or lowering of the liquid level in the pipette (14);
   wherein an advancing wetting angle of the liquid (22) is determined for a raising liquid level and/or a receding wetting angle of the liquid (22) is determined for a lowering liquid level.

2. The method of claim 1,
   wherein the pipette (14) is unused and has not been wetted with a liquid (22) before.

3. The method of claim 1 or 2, further comprising:

   before lowering the pipette (14) into the sample container (18) for a first time: aspirating and/or dispensing air into the pipette (14);
   determining a filter resistance of a filter (20) inside the pipette (14) from the pressure curve (40) during aspirating and/or dispensing air.

4. The method of claim 3, further comprising:
   determining an aspirating-dispensing asymmetry from the measured pressure values during aspirating and dispensing air.

5. The method of one of the previous claims, further comprising:

   after lowering the pipette (14) into the sample container (18) for the first time: dispensing air into the liquid (22) and generating at least one bubble in the liquid (22);
   determining a surface tension of the liquid (22) from the measured pressure curve (40) during generating the bubble.

6. The method of claim 5, further comprising:
   after generating the one or more bubbles, withdrawing the pipette (14) from the liquid (22) and dispensing air to remove liquid (22) from the pipette tip (16).

7. The method of one of the previous claims, further comprising:

waiting without aspirating and dispensing for a time period, while liquid (22) is in the pipette (14); determining a volatility of the liquid (22) from the measured pressure curve (40) during the time period.

8. The method of one of the previous claims, further comprising:

determining a viscosity of the liquid (22) from the pressure curve (40) during a time period with continuously raising and/or lowering of the liquid (22) in the pipette (14); and/or determining a density of the liquid (22) from the pressure curve (40) from two pressures of the pressure curve (40) at two times with two different known liquid levels.

9. The method of one of the preceding claims,

wherein the physical parameters are determined from a set of equations modelling the liquid (22) and the pipette (14); wherein the set of equations comprises the physical parameters of the liquid (22) and parameters of the pipette (14).

10. The method of claim 9,

wherein the liquid is an unknown liquid (22), the pipette is a second pipette (14) and the sample container is a second sample container (18); wherein the method further comprises:

pickup of a first pipette with the laboratory automation device (10), the first pipette being of equal structural form as the second pipette (14); lowering the first pipette into a first sample container with the laboratory automation device (10), the first sample container containing a known liquid with known physical parameters; aspirating and dispensing air and the known liquid with the laboratory automation device (10) in such a way, that a liquid level in the first pipette solely rises in a first step and solely lowers in a second step, such that an interior surface of the first pipette is wetted with the known liquid solely one time; during aspirating and dispensing air and the known liquid, measuring a pressure curve (40) in the first pipette and determining parameters of the first and second pipette from the pressure curve (40).

11. The method of one of the preceding claims, further comprising:

storing the physical parameters in a liquid class for the liquid (22); performing an assay procedure with the liquid (22) using the liquid class.

12. A computer program for determining physical parameters of a liquid, which computer program, when being executed by a processor and in a control device (34) of the laboratory automation device (10) as used in the method of claim 1, is adapted to carry out the steps of the method of one of the previous claims.

13. A computer-readable medium, in which a computer program according to claim 12 is stored.

14. A control device (34) of a laboratory automation device (10) as used in the method of claim 1, wherein the control device (34) is adapted for controlling a pump (26) and a pipetting arm (12) of the laboratory automation device (10), for receiving a pressure signal (40) from a pressure sensor (32) of the laboratory automation device (10) and for performing the method of one of claims 1 to 11.

15. A laboratory automation device (10), comprising:

a pipetting arm (12) for carrying a pipette (14); a pump (26) for changing a pressure in a volume (30) connected to the pipette (14); a pressure sensor (32) for pressure measurements in the volume (30) connected to the pipette (14); a control device (34) according to claim 14 for controlling the pump (26) and the pipetting arm (12) and for receiving a pressure signal (40) from the pressure sensor (32); wherein the control device (34) is adapted for performing the method of one of claims 1 to 11.

**Patentansprüche**

1. Verfahren zum Bestimmen physikalischer Parameter einer Flüssigkeit (22), die durch eine Laborautomatisierungsvorrichtung (10) angesaugt und/oder abgegeben werden soll, wobei das Verfahren umfasst:

Aufnehmen einer Pipette (14) mit der Laborautomatisierungsvorrichtung (10); Absenken der Pipette (14) in einen Probenbehälter (18) mit der Laborautomatisierungsvorrichtung (10), wobei der Probenbehälter (18) die Flüssigkeit (22) enthält;

Ansaugen und Abgeben von Luft und Flüssigkeit (22) mit der Laborautomatisierungsvorrichtung (10) in einer solchen Weise, dass ein Flüssigkeitsstand in der Pipette (14) in einem ersten Schritt ausschließlich ansteigt und in einem zweiten Schritt ausschließlich absinkt, dergestalt, dass eine Innenfläche der Pipette (14) nur ein einziges Mal mit Flüssigkeit benetzt wird; während des Ansaugens und Abgebens von Luft und Flüssigkeit (22), Messen einer Druckkurve (40) in der Pipette (14) und Bestimmen der physikalischen Parameter anhand der Druckkurve (40), wobei die physikalischen Parameter einen Benetzungswinkel der Flüssigkeit (22) und optional eine Oberflächenspannung und/oder optional eine Viskosität umfassen:

wobei der Benetzungswinkel der Flüssigkeit (22) anhand der Druckkurve (40) während eines Zeitraums mit kontinuierlichem Ansteigen und/oder Absinken des Flüssigkeitsstandes in der Pipette (14) bestimmt wird;
wobei ein Benetzungs-Fortschreitwinkel der Flüssigkeit (22) für einen ansteigenden Flüssigkeitsstand bestimmt wird und/oder ein Benetzungs-Rückzugswinkel der Flüssigkeit (22) für einen sinkenden Flüssigkeitsstand bestimmt wird.

2. Verfahren nach Anspruch 1,
wobei die Pipette (14) unbenutzt ist und zuvor nicht mit einer Flüssigkeit (22) benetzt wurde.

3. Verfahren nach Anspruch 1 oder 2, des Weiteren umfassend:

vor dem ersten Absenken der Pipette (14) in den Probenbehälter (18): Ansaugen und/oder Abgeben von Luft in die Pipette (14);
Bestimmen eines Filterwiderstands eines Filters (20) innerhalb der Pipette (14) anhand der Druckkurve (40) während des Ansaugens und/oder Abgebens von Luft.

4. Verfahren nach Anspruch 3, des Weiteren umfassend:
Bestimmen einer Ansaug-Abgabe-Asymmetrie anhand der gemessenen Druckwerte während des Ansaugens und Abgebens von Luft.

5. Verfahren nach einem der vorangehenden Ansprüche, des Weiteren umfassend:

nach dem erstmaligen Absenken der Pipette (14) in den Probenbehälter (18): Abgeben von Luft in die Flüssigkeit (22) und Generieren mindestens einer Blase in der Flüssigkeit (22);

Bestimmen einer Oberflächenspannung der Flüssigkeit (22) anhand der gemessenen Druckkurve (40) während des Generierens der Blase.

6. Verfahren nach Anspruch 5, des Weiteren umfassend:
nach dem Generieren der einen oder der mehreren Blasen, Herausziehen der Pipette (14) aus der Flüssigkeit (22) und Abgeben von Luft, um Flüssigkeit (22) aus der Pipettenspitze (16) zu entfernen.

7. Verfahren nach einem der vorangehenden Ansprüche, des Weiteren umfassend:
Warten einen Zeitraum lang ohne Ansaugen und Abgeben, während sich Flüssigkeit (22) in der Pipette (14) befindet;
Bestimmen einer Flüchtigkeit der Flüssigkeit (22) anhand der gemessenen Druckkurve (40) während des Zeitraums.

8. Verfahren nach einem der vorangehenden Ansprüche, des Weiteren umfassend:
Bestimmen einer Viskosität der Flüssigkeit (22) anhand der Druckkurve (40) während eines Zeitraums mit kontinuierlichem Ansteigen und/oder Absinken der Flüssigkeit (22) in der Pipette (14); und/oder
Bestimmen einer Dichte der Flüssigkeit (22) anhand der Druckkurve (40) anhand zweier Drücke der Druckkurve (40) zu zwei Zeiten mit zwei verschiedenen bekannten Flüssigkeitsständen.

9. Verfahren nach einem der vorangehenden Ansprüche,

wobei die physikalischen Parameter anhand eines Satzes von Gleichungen bestimmt werden, die die Flüssigkeit (22) und die Pipette (14) modellieren;
wobei der Satz von Gleichungen die physikalischen Parameter der Flüssigkeit (22) und Parameter der Pipette (14) umfasst.

10. Verfahren nach Anspruch 9,

wobei die Flüssigkeit eine unbekannte Flüssigkeit (22) ist, die Pipette eine zweite Pipette (14) ist, und der Probenbehälter ein zweiter Probenbehälter (18) ist;
wobei das Verfahren des Weiteren umfasst:

Aufnehmen einer ersten Pipette mit der Laborautomatisierungsvorrichtung (10), wobei die erste Pipette von gleicher struktureller Form wie die zweite Pipette (14) ist;
Absenken der ersten Pipette in einen ersten Probenbehälter mit der Laborautomatisierungsvorrichtung (10), wobei der erste Probenbehälter eine bekannte Flüssigkeit mit bekannten physikalischen Parametern enthält;

Ansaugen und Abgeben von Luft und der bekannten Flüssigkeit mit der Laborautomatisierungsvorrichtung (10) in einer solchen Weise, dass ein Flüssigkeitsstand in der ersten Pipette in einem ersten Schritt ausschließlich ansteigt und in einem zweiten Schritt ausschließlich absinkt, dergestalt, dass eine Innenfläche der ersten Pipette nur ein einziges Mal mit der bekannten Flüssigkeit benetzt wird;

während des Ansaugens und Abgebens von Luft und der bekannten Flüssigkeit, Messen einer Druckkurve (40) in der ersten Pipette und Bestimmen von Parametern der ersten und der zweiten Pipette anhand der Druckkurve (40).

11. Verfahren nach einem der vorangehenden Ansprüche, des Weiteren umfassend:

Speichern der physikalischen Parameter in einer Flüssigkeitsklasse für die Flüssigkeit (22); Durchführen eines Assay-Procederes mit der Flüssigkeit (22) unter Verwendung der Flüssigkeitsklasse.

12. Computerprogramm zum Bestimmen physikalischer Parameter einer Flüssigkeit, wobei das Computerprogramm, wenn es durch einen Prozessor und in einer Steuerungsvorrichtung (34) der Laborautomatisierungsvorrichtung (10) im Sinne des Verfahrens nach Anspruch 1 ausgeführt wird, dafür ausgelegt ist, die Schritte des Verfahrens nach einem der vorangehenden Ansprüche auszuführen.

13. Computerlesbares Medium, auf dem ein Computerprogramm nach Anspruch 12 gespeichert ist.

14. Steuerungsvorrichtung (34) einer Laborautomatisierungsvorrichtung (10) gemäß der Verwendung in dem Verfahren nach Anspruch 1, wobei die Steuerungsvorrichtung (34) dafür ausgelegt ist, eine Pumpe (26) und einen Pipettierarm (12) der Laborautomatisierungsvorrichtung (10) zu steuern, ein Drucksignal (40) von einem Drucksensor (32) der Laborautomatisierungsvorrichtung (10) zu empfangen, und das Verfahren nach einem der Ansprüche 1 bis 11 durchzuführen.

15. Laborautomatisierungsvorrichtung (10), umfassend:

einem Pipettierarm (12) zum Tragen einer Pipette (14);
eine Pumpe (26) zum Verändern eines Drucks in einem mit der Pipette (14) verbundenen Volumen (30);
einen Drucksensor (32) für Druckmessungen in dem mit der Pipette (14) verbundenen Volumen (30);
eine Steuerungsvorrichtung (34) nach Anspruch 14 zum Steuern der Pumpe (26) und des Pipettierarms (12) und zum Empfangen eines Drucksignals (40) von dem Drucksensor (32);
wobei die Steuerungsvorrichtung (34) dafür ausgelegt ist, das Verfahren nach einem der Ansprüche 1 bis 11 durchzuführen.

## Revendications

1. Procédé pour déterminer des paramètres physiques d'un liquide (22) à aspirer et/ou distribuer par un dispositif d'automatisation de laboratoire (10), le procédé comprenant de :

saisir une pipette (14) avec le dispositif d'automatisation de laboratoire (10) ;
abaisser la pipette (14) dans un contenant à échantillon (18) avec le dispositif d'automatisation de laboratoire (10), le contenant à échantillon (18) contenant le liquide (22) ;
aspirer et distribuer de l'air et du liquide (22) avec le dispositif d'automatisation de laboratoire (10) de telle manière qu'un niveau de liquide dans la pipette (14) monte exclusivement dans une première étape et descend exclusivement dans une seconde étape, de telle sorte qu'une surface intérieure de la pipette (14) est mouillée une seule fois avec du liquide ;
pendant l'aspiration et la distribution d'air et du liquide (22), mesurer une courbe de pression (40) dans la pipette (14) et déterminer les paramètres physiques à partir de la courbe de pression (40), les paramètres physiques comprenant un angle de mouillage du liquide (22) et facultativement une tension superficielle et/ou facultativement une viscosité ;
dans lequel l'angle de mouillage du liquide (22) est déterminé à partir de la courbe de pression (40) pendant une période de temps avec une montée et/ou une descente continues du niveau de liquide dans la pipette (14) ;
dans lequel un angle de mouillage avançant du liquide (22) est déterminé pour un niveau de liquide qui monte et/ou un angle de mouillage reculant du liquide (22) est déterminé pour un niveau de liquide qui descend.

2. Procédé selon la revendication 1,
dans lequel la pipette (14) n'est pas utilisée et n'a pas été préalablement mouillée avec un liquide (22).

**3.** Procédé selon la revendication 1 ou 2, comprenant en outre de :

> avant de faire descendre la pipette (14) dans le contenant à échantillon (18) pour la première fois : aspirer et/ou distribuer de l'air dans la pipette (14) ;
> déterminer une résistance de filtre d'un filtre (20) à l'intérieur de la pipette (14) à partir de la courbe de pression (40) pendant l'aspiration et/ou la distribution d'air.

**4.** Procédé selon la revendication 3, comprenant en outre de :
déterminer une asymétrie d'aspiration-distribution à partir des valeurs de pression mesurées pendant l'aspiration et la distribution d'air.

**5.** Procédé selon l'une des revendications précédentes, comprenant en outre de :

> après avoir abaissé la pipette (14) dans le contenant à échantillon (18) pour la première fois : distribuer de l'air dans le liquide (22) et générer au moins une bulle dans le liquide (22) ;
> déterminer une tension superficielle du liquide (22) à partir de la courbe de pression (40) mesurée pendant la génération de la bulle.

**6.** Procédé selon la revendication 5, comprenant en outre de :
après avoir généré les une ou plusieurs bulles, extraire la pipette (14) du liquide (22) et distribuer de l'air pour retirer le liquide (22) de l'embout de pipette (16).

**7.** Procédé selon l'une des revendications précédentes, comprenant en outre de :

> attendre sans aspirer et distribuer pendant un certain temps, alors que le liquide (22) est dans la pipette (14) ;
> déterminer une volatilité du liquide (22) à partir de la courbe de pression (40) mesurée pendant la période de temps.

**8.** Procédé selon l'une des revendications précédentes, comprenant en outre de :

> déterminer une viscosité du liquide (22) à partir de la courbe de pression (40) pendant une période de temps avec une montée et/ou une descente continues du liquide (22) dans la pipette (14) ; et/ou
> déterminer une densité du liquide (22) à partir de la courbe de pression (40) à partir de deux pressions de la courbe de pression (40) à deux instants avec deux différents niveaux de liquide connus.

**9.** Procédé selon l'une des revendications précédentes,

> dans lequel les paramètres physiques sont déterminés à partir d'un ensemble d'équations modélisant le liquide (22) et la pipette (14) ;
> dans lequel l'ensemble d'équations comprend les paramètres physiques du liquide (22) et des paramètres de la pipette (14).

**10.** Procédé selon la revendication 9,

> dans lequel le liquide est un liquide inconnu (22), la pipette est une seconde pipette (14) et le contenant à échantillon est un second contenant à échantillon (18) ;
> dans lequel le procédé comprend en outre de :

>> saisir une première pipette avec le dispositif d'automatisation de laboratoire (10), la première pipette étant de forme structurelle égale à la seconde pipette (14) ;
>> abaisser la première pipette dans un premier contenant à échantillon avec le dispositif d'automatisation de laboratoire (10), le premier contenant à échantillon contenant un liquide connu avec des paramètres physiques connus ;
>> aspirer et distribuer de l'air et le liquide connu avec le dispositif d'automatisation de laboratoire (10) de telle manière qu'un niveau de liquide dans la première pipette monte exclusivement dans une première étape et descend exclusivement dans une seconde étape, de telle sorte qu'une surface intérieure de la première pipette est mouillée une seule fois avec le liquide connu ;
>> lors de l'aspiration et de la distribution d'air et du liquide connu, mesurer une courbe de pression (40) dans la première pipette et déterminer des paramètres des première et seconde pipettes à partir de la courbe de pression (40).

**11.** Procédé selon l'une des revendications précédentes, comprenant en outre de :

> stocker les paramètres physiques dans une classe de liquide pour le liquide (22) ;
> mettre en œuvre une procédure d'analyse avec le liquide (22) en utilisant la classe de liquide.

**12.** Programme informatique pour déterminer des paramètres physiques d'un liquide, lequel programme informatique, lorsqu'il est exécuté par un processeur et dans un dispositif de commande (34) du dispositif d'automatisation de laboratoire (10) tel qu'utilisé dans le procédé de la revendication 1, est adapté pour mettre en œuvre les étapes du procédé selon l'une des revendications précédentes.

13. Support lisible par ordinateur, sur lequel est stocké un programme informatique selon la revendication 12.

14. Dispositif de commande (34) d'un dispositif d'automatisation de laboratoire (10) tel qu'utilisé dans le procédé selon la revendication 1, dans lequel le dispositif de commande (34) est adapté pour commander une pompe (26) et un bras de pipetage (12) du dispositif d'automatisation de laboratoire (10), pour recevoir un signal de pression (40) provenant d'un capteur de pression (32) du dispositif d'automatisation de laboratoire (10) et pour mettre en œuvre le procédé selon l'une des revendications 1 à 11.

15. Dispositif d'automatisation de laboratoire (10), comprenant :

    un bras de pipetage (12) pour porter une pipette (14) ;
    une pompe (26) pour faire varier une pression dans un volume (30) relié à la pipette (14) ;
    un capteur de pression (32) pour des mesures de pression dans le volume (30) relié à la pipette (14) ;
    un dispositif de commande (34) selon la revendication 14 pour commander la pompe (26) et le bras de pipetage (12) et pour recevoir un signal de pression (40) provenant du capteur de pression (32) ;
    dans lequel le dispositif de commande (34) est adapté pour mettre en œuvre le procédé selon l'une des revendications 1 à 11.

# Fig. 1

# Fig. 2

**EP 4 386 389 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2022026455 A1